# EUROPEAN PATENT APPLICATION

(11) **EP 3 868 403 A1**
(43) Date of publication of application: **25.08.2021**
(21) Application number: 19937362.2
(22) Date of filing: 24.12.2019
(51) Int. Cl.: A61K 39/395, A61P 37/02

(54) **TACI-FC FUSION PROTEIN AND USE THEREOF**

(71) Applicant: RemeGen Co., Ltd., Yantai, Shandong 264006 (CN)
(72) Inventor: FANG, Jianmin, Yantai Shandong 264006 (CN); WANG, Wenxiang, Yantai Shandong 264006 (CN); LI, Lin, Yantai Shandong 264006 (CN)
(74) Representative: HGF
(86) International application number: PCT/CN2019/128099
(87) International publication number: WO 2021/128027

(57) **Abstract**

The present invention relates to an optimized TACI-Fc fusion protein and use of the same in the manufacture of a medicament for treating systemic lupus erythematosus, and a dosage regimen or method for treating systemic lupus erythematosus using the TACI-Fc fusion protein.

SEQUENCE LISTING

<110>  REMEGEN CO., LTD.

<120>  TACI-FC FUSION PROTEIN AND USE THEREOF
<130>  1

<160>  1

<170>  PatentIn version 3.5

<210>  1
<211>  333
<212>  PRT
<213>  Artificial sequence

<220>
<223>  The sequence of the TACI-Fc fusion protein

<400>  1

Ser Arg Val Asp Gln Glu Glu Arg Phe Pro Gln Gly Leu Trp Thr Gly
1               5                   10                  15

Val Ala Met Arg Ser Cys Pro Glu Glu Gln Tyr Trp Asp Pro Leu Leu
            20                  25                  30

Gly Thr Cys Met Ser Cys Lys Thr Ile Cys Asn His Gln Ser Gln Arg
        35                  40                  45

Thr Cys Ala Ala Phe Cys Arg Ser Leu Ser Cys Arg Lys Glu Gln Gly
    50                  55                  60

Lys Phe Tyr Asp His Leu Leu Arg Asp Cys Ile Ser Cys Ala Ser Ile
65                  70                  75                  80

Cys Gly Gln His Pro Lys Gln Cys Ala Tyr Phe Cys Glu Asn Lys Leu
                85                  90                  95

Arg Ser Pro Val Asn Leu Pro Pro Glu Leu Asp Lys Thr His Thr Cys
            100                 105                 110

Pro Pro Cys Pro Ala Pro Glu Ala Glu Gly Ala Pro Ser Val Phe Leu
        115                 120                 125

Phe Pro Pro Lys Pro Lys Asp Thr Leu Met Ile Ser Arg Thr Pro Glu
    130                 135                 140

Val Thr Cys Val Val Val Asp Val Ser His Glu Asp Pro Glu Val Lys
145                 150                 155                 160

Phe Asn Trp Tyr Val Asp Gly Val Glu Val His Asn Ala Lys Thr Lys
                165                 170                 175

Pro Arg Glu Glu Gln Tyr Asn Ser Thr Tyr Arg Val Val Ser Val Leu
            180                 185                 190

Thr Val Leu His Gln Asp Trp Leu Asn Gly Lys Glu Tyr Lys Cys Lys
        195                 200                 205

Val Ser Asn Lys Ala Leu Pro Ser Ser Ile Glu Lys Thr Ile Ser Lys
    210                 215                 220

Ala Lys Gly Gln Pro Arg Glu Pro Gln Val Tyr Thr Leu Pro Pro Ser
225                 230                 235                 240

Arg Asp Glu Leu Thr Lys Asn Gln Val Ser Leu Thr Cys Leu Val Lys
                245                 250                 255

Gly Phe Tyr Pro Ser Asp Ile Ala Val Glu Trp Glu Ser Asn Gly Gln
            260                 265                 270

Pro Glu Asn Asn Tyr Lys Thr Thr Pro Pro Val Leu Asp Ser Asp Gly
        275                 280                 285

Ser Phe Phe Leu Tyr Ser Lys Leu Thr Val Asp Lys Ser Arg Trp Gln
    290                 295                 300

Gln Gly Asn Val Phe Ser Cys Ser Val Met His Glu Ala Leu His Asn
305                 310                 315                 320

His Tyr Thr Gln Lys Ser Leu Ser Leu Ser Pro Gly Lys
                325                 330

## Description

### FIELD

The present invention relates to the field of a B lymphocyte stimulator receptor-antibody fusion protein for treatment of autoimmune diseases, and more specifically, to an optimized TACI-Fc fusion protein and use of the same in the manufacture of a medicament for treating systemic lupus erythematosus, and a dosage regimen for treating autoimmune diseases using the same.

### BACKGROUND

Systemic lupus erythematosus (SLE) is a chronic, multi-system-involved autoimmune disease, characterized by abnormal activation of B lymphocytes and abnormal differentiation into plasma cells and memory effector cells to produce a large amount of autoantibodies. It seriously threatens human health. At present, there are lesser specific therapeutics for SLE clinically and conventional treatment of SLE is mainly based on glucocorticoids and immunosuppressants, and corticosteroids are the basis of all treatment options. Cyclophosphamide is currently an important drug for the treatment of severe lupus nephritis, but its clinical use is limited by the large adverse reactions. In addition, hydroxychloroquine approved by the FDA in 1955 has unsatisfactory therapeutic effect.

Studies have shown that BLyS knockout mice have reduced mature B lymphocytes and reduced immunoglobulins, while BLyS transgenic mice show lupus-like manifestations such as severe B lymphocyte proliferation, high titers of anti-dsDNA autoantibodies, proteinuria, and deposition of immune complexes in the kidneys. In two SLE mouse models MRL/1pr and NZB/WF1, at the onset of the disease, serum BLyS levels are increased, which is related to kidney damage, and blocking the effect of BLyS could significantly improve the survival of mice with lupus-like symptoms. In the serum and plasma of some SLE patients, there are significantly higher level and biological activity of BLyS than in the normal people. The results of animal experiments and human in vitro experiments suggest that overexpression of BLyS is one of the important mechanisms involved in the pathogenesis of SLE. Therefore, targeting BLyS and eliminating B lymphocytes has become a very promising treatment strategy for SLE.

Currently, biologics targeting BLyS for the treatment of SLE have become a research hotspot. BLyS antagonists for treatment of SLE may include BLyS monoclonal antibody, fusion protein composed of BLyS receptor and IgG-Fc segment, BLyS mimics that can bind to BAFF-R but cannot initiate signal transduction, and non-agonistic BLyS receptor antibodies, etc.

B lymphocyte stimulators (BLyS) is a new member of the TNF superfamily discovered in 1999, and mainly secreted by monocytes, macrophages, dendritic cells and neutrophils. BLyS exerts the functions of regulating the maturation of B lymphocytes, promoting the conversion of immunoglobulin classes, assisting T cell activation and participating in autoimmunity by binding to its corresponding receptors. BLyS is composed of 285 amino acids and is expressed on the cell surface as a type II transmembrane protein, and the 17 kb soluble fragment released after shearing has biological activity and participates in the circulation as a homotrimer. There are currently three known BLyS receptors: BCMA (B cell maturation antigen), BAFF-R (BAFF receptor, BR3), and TACI (transmembrane activator and calcium modulator and cyclophilin ligand interactor, TACI). BAFF-R is a specific receptor for BLyS, while TACI and BCMA can also bind to APRIL in addition to binding to BLyS. The three receptors of BLyS play different roles when binding to BLyS. The roles of BLyS in promoting the maturation of B cell in the transitional period of mice, prolonging the life span of mature B cells in mice and humans, and assisting in T cell activation are mainly achieved through BAFF-R. TACI receptors have a dual effect on the activation of mouse B cells. On the one hand, TACI-deficient mice exhibit increased peripheral B cells, increased antigen synthesis, autoimmune and even lethal lymphoid proliferation, suggesting that TACI is an inhibitory receptor of BLyS. On the other hand, TACI participates in the immune response of T-cell independent antigen (TI). BCMA has a function of maintaining the survival of plasmablasts and promoting the antigen presentation of mature B cells. Since TACI receptor has a strong affinity for both BLyS and APRIL, TACI-Fc fusion protein is preferred compared with the other two fusion proteins, and if preferred for drug structure.

TACI was first discovered by scientists Von Bulow and Bram at St. Jude Children's Hospital, a well-known American academic institution, but later studies found that the natural sequence of the extracellular region of TACI has the problem of vulnerable degradation, and is not suitable for drug manufacture. Since then, several companies have made improvements to the original TACI molecule, including the famous American biotechnology companies Genentech, Amgen and ZymoGenetics. At present, the TACI fusion protein Atacicept developed by ZymoGenetics and Merck Serono in Switzerland has undergone clinical trials for SLE, rheumatoid arthritis, lymphoma and other diseases. A Phase III clinical trial (NCT00624338) for the efficacy and safety of Atacicept for the prevention of moderate to severe SLE showed that compared with the placebo group, there was no significant difference between the 75mg dose of Atacicept and the placebo group (58% and 60%; OR 0.89 (0.48, 1.67), p=0.724); and the 150 mg dose of Atacicept showed efficacy (43% and 60%; OR 0.49 (0.26, 0.92), p=0.027). However, the death of two subjects due to pneumonia and pulmonary hemorrhage suggested safety problems. From the clinical results, low-dose of Atacicept had no efficacy, while high-dose of Atacicept led to serious safety problems.

Internationally, the development of SLE drugs is not progressing smoothly. Until March 9, 2011, the Blys-specific inhibitor belimumab developed by GSK became the first new drug approved by the FDA for the treatment of systemic lupus erythematosus in more than 50 years, opening a new chapter in the clinical treatment of SLE. This drug is currently the only Blys antibody, with global sales of 473 million pounds in 2018. The subcutaneous injection formulation of belimumab in a dose of 200 mg once a week has been approved by the FDA for use in adult patients with active, autoantibody-positive systemic lupus erythematosus (SLE) who are receiving standard treatment. Clinical trials have shown that belimumab can significantly reduce the 52-week systemic lupus erythematosus disease activity index and reduce disease recurrence in SLE patients. The results showed that the proportion of patients who achieved remission through belimumab treatment gradually increased over time, and the total remission rate at year 10 was 65.2% (n=126). In the patients with baseline prednisone dose more than 7.5 mg/d, 32.6% (14/43) of them reduced the dose to ≤7.5 mg/d at year 10; and in the patients with baseline prednisone dose ≤7.5 mg/d, 9.5% (9/95) of them increased the dose to more than 7.5 mg/d at year 10.

CN101323643B and its patent family member US8193316B2 found the reasons for the degradation of TACI natural sequence, and then constructed an optimized TACI-Fc fusion protein by using genetic engineering (the entire contents of CN101323643B and US8193316B2 are incorporated herein by reference), and specifically disclosed a fusion protein consisting of truncated TACI and the Fc region of immunoglobulin. The test results show that the optimized TACI-Fc fusion protein has a better biological activity for inhibiting autoimmune diseases, and has potential treatment prospects for autoimmune diseases such as SLE, rheumatoid arthritis, neuromyelitis optica (NMO), neuromyelitis optica spectrum disorders (NMOSD), etc. In addition, CN102085368B discloses the use of the optimized TACI-Fc fusion protein in the manufacture of a medicament for the treatment of systemic lupus erythematosus (the entire content of CN102085368B is incorporated herein by reference), and it is proved to have good tolerance and safety through previous animal experiments.

The current options for the treatment of systemic lupus erythematosus are very limited, and there is a need to develop a new treatment with better curative effect and no serious safety risk. The present invention addresses this need.

### SUMMARY

The present invention relates to a fusion protein consisting of truncated TACI and optimized immunoglobulin Fc via sequence optimization with reduced ADCC and CDC effects, which has excellent biological activity and safety for treating human autoimmune system diseases. The fusion protein inhibits the two biologically active molecules BLyS and APRIL to block the proliferation of B lymphocytes and the maturation of T lymphocytes, achieving the purpose of treating autoimmune diseases such as systemic lupus erythematosus and rheumatoid arthritis. The fusion protein comprises amino acids 13-118 of the extracellular domain of TACI and the Fc region of human immunoglobulin. The amino acids 13-118 of the extracellular domain of TACI is shown in amino acids 1-106 of SEQ ID NO: 1, or has 80%, 85%, 90%, 91%, 92%, 93%, 94%, 95%, 96%, 97%, 98%, 99% or more homology with amino acids 1-106 of SEQ ID NO: 1. The Fc region of human immunoglobulin is shown in amino acids 107-333 of SEQ ID NO: 1, or has 80%, 85%, 90%, 91%, 92%, 93%, 94%, 95%, 96%, 97%, 98%, 99% or more homology with amino acids 107-333 of SEQ ID NO: 1.

In some embodiments, the amino acid sequence of the TACI-Fc fusion protein is shown in SEQ ID NO: 1. The protein consists of amino acids 13-118 of the extracellular region of TACI and an optimized Fc fragment via optimization with reduced ADCC and CDC effects.

In some embodiments, the patients to be treated have moderate to severe systemic lupus erythematosus.

In the TACI-Fc fusion protein shown in SEQ ID NO: 1, in order to avoid antibody-dependent cell-mediated toxicity (ADCC) effects due to membrane-bound BLyS or A Proliferation-Inducing Ligand (APRIL), the sequence of the Fc fragment derived from IgG1 is optimized. The amino acids 120-123 in the CH2 region of the Fc fragment are mutated from Leucine (L)-Leucine (L)-Glycine (G)-Glycine (G) to Alanine (A)-Glutamic acid (E)-Glycine (G)-Alanine (A), to reduce the affinity to the Fcy receptor. In addition, the amino acids 216-217 in the CH2 region of the Fc fragment is also mutated from alanine (A)-proline (P) to serine (S)-serine (S), to reduce complement binding or fixation, thereby reducing complement-dependent cytotoxicity (CDC) effect.

The sequence of SEQ ID NO: 1 is as follows:

001 SRVDQEERFP QGLWTGVAMR SCPEEQYWDP LLGTCMSCKT ICNHQSQRTC

051 AAFCRSLSCR KEQGKFYDHL LRDCISCASI CGQHPKQCAY FCENKLRSPV

101 NLPPELDKTH TCPPCPAPEA EGAPSVFLFP PKPKDTLMIS RTPEVTCVVV

151 DVSHEDPEVK FNWYVDGVEV HNAKTKPREE QYNSTYRVVS VLTVLHQDWL

201 NGKEYKCKVS NKALPSSIEK TISKAKGQPR EPQVYTLPPS RDELTKNQVS

251 LTCLVKGFYP SDIAVEWESN GQPENNYKTT PPVLDSDGSF FLYSKLTVDK

301 SRWQQGNVFS CSVMHEALHN HYTQKSLSLS PGK

In liquid preparations, two TACI-Fc fusion protein monomers can form a double-stranded structure due to the formation of interchain disulfide bonds in the Fc hinge region.

The TACI-Fc fusion protein of the present invention can be prepared with a pharmaceutically acceptable carrier into a medicament for the treatment of systemic lupus erythematosus (SLE) by conventional methods in the field.

The medicament of the present invention can be made into various clinically acceptable dosage forms, including but not limited to lyophilized preparations or liquid preparations administered by manners including subcutaneous injection, intraperitoneal injection, intramuscular injection, or intravenous injection, and preferably by subcutaneous injection.

In an aspect, the TACI-Fc fusion protein preparation of the present invention is preferably administered by subcutaneous injection at a dose greater than or equal to 60 mg per administration, or 70 mg per administration, or 80 mg per administration. More preferably, the dose is about 50-240 mg per administration, 60-240 mg per administration, 70-240 mg per administration, or 80-240 mg per administration, and more preferably, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 190 mg, 195 mg, 200 mg, 205 mg, 210 mg, 215 mg, 220 mg, 225 mg, 230 mg, 235 mg , or 240 mg per administration.

In certain embodiments, the TACI-Fc fusion protein of the present invention is administered to the patient once every 6-10 days to 11-14 days.

In certain embodiments, the TACI-Fc fusion protein of the present invention is administered to the patient once every week, every two weeks, every three weeks, or every four weeks.

As used herein, "therapeutically effective amount" or "effective amount" refers to a dose sufficient to show the benefit to the patient being administered. The actual amount administered, as well as the rate and time course of administration, will depend on the condition of the individual being treated and the severity of disease. The prescription of treatment (such as the decision on dosage, etc.) is ultimately decided by general practitioners and other doctors, usually considering the disease to be treated, the individual condition of the patient, the delivery site, the manner of administration, and other factors known to the doctor.

In certain embodiments, the TACI-Fc fusion protein of the present invention is administered to the patient for consecutive 12 weeks, 24 weeks, 36 weeks, 48weeks, 60 weeks, 72 weeks.

In some embodiments, the recombinant TACI-Fc fusion protein of the present invention has an amino acid sequence as shown in SEQ ID NO: 1.

In some embodiments, the nucleotide sequence of the recombinant TACI-Fc fusion protein of the present invention encodes the amino acid sequence as shown in SEQ ID NO: 1.

In some embodiments, the present invention provides a vector, being capable of expressing the above fusion protein.

In some embodiments, the present invention provides a pharmaceutical composition, comprising the above recombinant TACI-Fc fusion protein and a pharmaceutically acceptable carrier.

In some embodiments, the pharmaceutical composition/fusion protein of the present invention can be used to treat autoimmune diseases, including but not limited to systemic lupus erythematosus (SLE), rheumatoid arthritis (RA), neuromyelitis optica (NMO) and neuromyelitis optica spectrum disorders (NMOSD).

In some embodiments, the systemic lupus erythematosus treated with the pharmaceutical composition/fusion protein of the present invention is mild to moderate systemic lupus erythematosus or moderate to severe systemic lupus erythematosus.

The further structural optimization to the TACI-Fc fusion protein disclosed in US8193316B2 obtains TACI-Fc fusion protein with greatly improved safety, which can be administered to patient at higher dose without serious safety risks that may endanger the lives of patients. In large-scale clinical trials, we have found that compared with Atacicept, when being administered at an effective dose, the TACI-Fc fusion protein of the present invention can minimize the cytotoxicity of the fusion protein, reduces the side reactions brought to patients during the treatment and has no serious safety risks, and unexpectedly restore the fertility ability of some patients by treating the patients with systemic lupus erythematosus, achieving unexpected technical effects.

**BRIEF DESCRIPTION OF DRAWINGS**
Figure 1: The overlay of the ADCC effect curve (effector-target ratio (E:T) = 10:1) of TACI-Fc fusion protein (SEQ ID NO.1) (green, solid) / rituximab (red, hollow).
Figure 2: The overlay of the ADCC effect curve (effector-target ratio (E:T) =20:1) of TACI-Fc fusion protein (SEQ ID NO.1) (green, solid) / rituximab (red, hollow).

### DETAILED DESCRIPTION

### Example 1. Comparison test of the ADCC effect of TACI-Fc fusion protein (SEQ ID NO. 1)

### 1.1 Experimental materials

**Table 1**

| Materials | Manufacturer | Batch No |
|---|---|---|
| RPMI 1640 medium | Gibco | A10491 |
| Rituximab | Roche | 10001031283948 |
| 96-well cell plate | Costar | 3599 |
| PBMC cells | Hemacare | PB009C-1 |
| LDH detection kit | Roche | 1164793001 |
| FBS | Excellbio | FND500 |
| TritonX-100 | Solarbio | T8200 |

### 1.2 Experimental principle

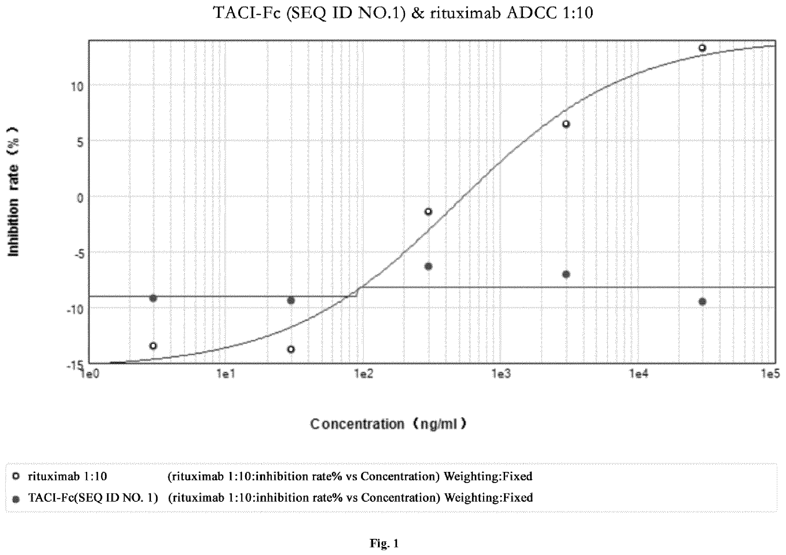

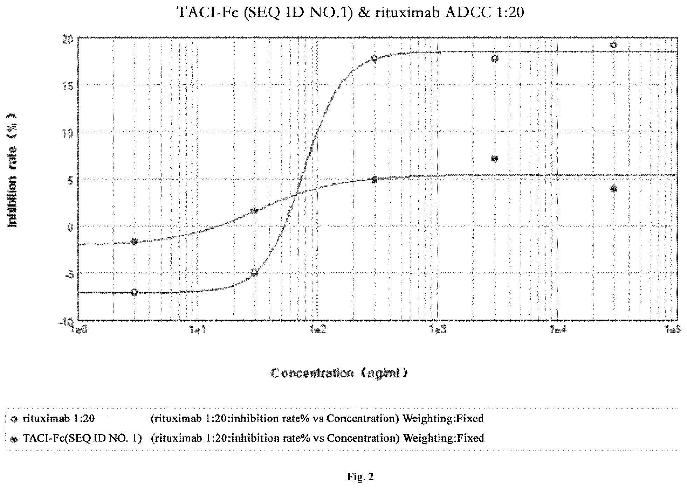

Raji cells express APRIL on the surface, and TACI is the co-receptor of APRIL and BLyS. Therefore, APRIL on the surface of Raji cells can bind to the TACI terminal of TACI-Fc fusion protein (SEQ ID NO: 1). If the Fc terminal of the TACI-Fc fusion protein (SEQ ID NO: 1) has a binding site of crystallizable fragment γ receptor (Fc γ receptor, FcyR), it can bind to the FcyR of effector cell and trigger the ADCC effect. Rituximab was used as a positive control in the test because rituximab is a Cluster of Differentiation 20 (CD20) monoclonal antibody capable of inducing the ADCC effect. Raji cell expresses CD20 on the surface, and therefore, rituximab can bind to CD20 on the surface of Raji cells to trigger ADCC effects. The experimental results are shown in Figure 1 and Figure 2: The control rituximab induces ADCC effect, and the inhibition rate is concentration-dependent. Compared with the control, TACI-Fc fusion protein does not induce ADCC effect at each concentration.

### Example 2: Comparison test of the CDC effect of TACI-Fc fusion protein

### 2.1 Experimental materials

**Table 2**

| Materials | Manufacturer | Batch No |
|---|---|---|
| RPMI 1640 medium | Gibco | A10491 |
| Rituximab | Roche | 10001031283948 |
| Human serum complement | Donor | |
| Cell titer-gloluminescent cell viability assay | Promega | G7572 |
| Transparent 96-well plate with white bottom | Costar | 3610 |

### 2.2 Experimental principle

APRIL on the surface of Raji cells can bind to the TACI terminal of TACI-Fc fusion protein (SEQ ID NO: 1). If the Fc terminal of the TACI-Fc fusion protein (SEQ ID NO: 1) has a complement binding site, it can trigger the CDC effect. Rituximab was used as a positive control in the test because rituximab can induce the CDC effect. Raji cell expresses CD20 on the surface, and therefore, rituximab can bind to CD20 on the surface of Raji cells to trigger CDC effects. Monoclonal antibody that does not bind to Raji cells was used as a negative control.

### 2.3 Experimental procedure

After adjusting the concentration of Raji cells to 10,000 cells/well, the cells were added a 96-well plate and incubated in a CO₂ incubator for 20 minutes. Then, diluted series of samples of different concentration gradients were added and then the cells were incubated in the CO₂ incubator for 20 minutes. Then, 10% normal human serum complement was added and then the cells were incubated in the CO₂ incubator for 2 hours. After 10 minutes of color development using the ATP method, a microplate reader was used for reading. After subtracting the serum control value, GraphPad Prism 7.00 was used to make a 4-PL curve with the concentration (ng/ml) of lg on the abscissa and the inhibition rate (%) on the ordinate. The experimental results are as follows:

**Table 3. Results of the he CDC effect**

| Final concentration (ng/ml) | Inhibition rate% | | |
|---|---|---|---|
| | Rituximab | TACI-Fc fusion protein (SEQ ID NO: 1) | Negative control |
| 30000 | 38.17 | 4.88 | 5.60 |
| 3000 | 24.65 | 2.01 | 9.63 |
| 300 | 11.85 | 9.12 | -0.14 |
| 30 | 4.83 | 1.85 | -0.76 |
| 3 | -0.68 | 2.27 | -0.66 |

Experimental results:
1. Compared with the cell control group, each serum complement and drug control group has no obvious killing effect.
2. The positive control group (10% serum complement-rituximab) has a highest inhibition rate of 40%, and has a significant concentration-dependent effect.
3. The administration group (10% serum complement-TACI-Fc fusion protein (SEQ ID NO.1)) has a highest inhibition rate less than 10% and has no concentration-dependent effect.
4. The negative control group (10% serum complement-RC48) has a highest inhibition rate less than 10% and has no concentration-dependent effect.

It can be seen that rituximab can induce a CDC effect, and the inhibition rate is obviously concentration-dependent, while the TACI-Fc fusion protein (SEQ ID NO.1) basically induce no CDC effect.

### Example 3: The clinical effect trials of the treatment for patients with systemic lupus erythematosus

1. The experimental purpose is to preliminarily evaluate the safety and effectiveness of TACI-Fc fusion protein (with amino acid sequence shown in SEQ ID NO: 1) in standard combination therapy compared with placebo in standard combination therapy in the treatment of moderate to severe SLE patients, and analyze the dose-effect relationship to provide dose basis for follow-up clinical trials. The effective count of subjects in this experiment is 249 and the administrations in those showing no effect are stopped according to the dosing schedule.

### 2. Inclusion criteria

1. Patients diagnosed with active SLE who meet at least 4 of the 11 SLE standards revised by the American College of Rheumatology in 1997;
2. Subject with age from 18 to 65 years old, both male and female, no gender ratio limit;
3. Inpatients or outpatients with good compliance, and providing an informed consent form before the trial.
4. Subject with SELENA-SLEDAI score ≥ 8 points during the screening period. For those with low complement and/or anti-ds-DNA antibody score, the score for the clinical symptoms of SELENA-SLEDAI (except for low complement and/or anti-ds-DNA antibody) should be ≥6 points;
5. Subjects with positive in autoantibody serological test. Positive is defined as ANA positive and/or anti-double-stranded DNA antibody positive results defined by the clear reference value range of the research center laboratory, and critical values are not accepted;
6. Subjects with stable standard treatment regimen for at least 30 days before the first day (ie, the day when the test drug is administered fist). The standard treatment regimen refers to the stable use of any one of the following (alone or in combination): corticosteroids, antimalarials, non-steroidal anti-inflammatory drugs (NSAIDs), other immunosuppressants or immunomodulators including azathioprine, mycophenolate (including mycophenolate mofetil, sodium mycophenolate), cyclophosphamide, methotrexate, leflunomide, tacrolimus, cyclosporine.

### 3. Exclusion criteria

1. Subjects with severe lupus nephritis (defined as urine protein>6g/24 hours or serum creatinine>2.5 mg/dL or 221 umol/L) or required hemodialysis or received high-dose corticosteroids (prednisone>100mg/day or equivalent) ≥14 days in the last 2 months;
2. Subjects with central nervous system disease caused by SLE or not caused by SLE (including epilepsy, psychosis, organic brain syndrome, cerebrovascular accident, encephalitis, central nervous system vasculitis) in the last 2 months;
3. Subjects with severe diseases and disease history in the heart, liver, kidney and other important organs, blood, endocrine system; evaluation criteria for severity includes: a. ALT or AST ≥ 2 times the upper limit of normal range; b. endogenous creatinine clearance rate <30 mL/min; c. white blood cell count<2.5 × 10⁹/L; d. hemoglobin<85g/L; e. platelet count<50×10⁹/L.
4. Subjects currently suffering from active hepatitis or having severe liver disease or disease history. Hepatitis B: patients having positive HBsAg will be excluded. For subject having negative HBsAg and positive anti-HBc antibodies, they will be classified according to the HBV-DNA test, in which patients having positive HBV-DNA will be excluded and not allowed to participate in the study, and patients having negative HBV-DNA may be allowed to participate in the study. Hepatitis C: patients with positive hepatitis C antibodies will be excluded.
5. Patients with immunodeficiency, uncontrolled severe infection, and active or recurrent peptic ulcer;
6. Pregnant women, breastfeeding women, and men or women who have planned fertility in the next 12 months;
7. Allergic reactions: subject with a history of allergies to human-derived biological products;
8. Those who received live vaccines in the last month;
9. Those who have participated in any clinical trial 28 days before the initial screening and/or within 5 times the half-life of the study compound (whichever is longer);
10. Those who received B-cell targeted therapies, such as Rituxan or Epalizumab within one year;
11. Those who received tumor necrosis factor inhibitors and interleukin receptor blockers within one year;
12. Those who have received intravenous gamma globulin (IVIG), prednisone ≧100 mg/d for more than 14 days or undergo plasma exchange within one month;
13. Those having active infections (such as herpes zoster, HIV infection, active tuberculosis, etc.) during the screening period;
14. Mental patients with depression or suicidal thoughts;
15. Subjects who are considered unsuitable to participate in this trial by the researcher.

### IV Grouping

Treatment group: recombinant TACI-Fc fusion protein (with amino acid sequence shown in SEQ ID NO: 1) freeze-dried powder preparation; strength: 80 mg per injection; subcutaneous injection, dosage: 80 mg, 160 mg or 240 mg per administration (the indication of 80 mg, 160 mg, or 240 mg refers to the mass of the active ingredient TACI-Fc fusion protein in the lyophilized powder preparation), administered once a week for consecutive 48 weeks.

Control group: simulant of the recombinant TACI-Fc fusion protein lyophilized powder, administered by subcutaneous once a week for consecutive 48 weeks.

The specific grouping is as follows:

**Table 4 Distribution of cases in each center (randomized cases)**

| | RC18 240mg (N=62) | RC18 160mg (N=63) | RC1880mg (N=62) | Placebo (N=62) | Total (N=249) |
|---|---|---|---|---|---|
| Summary | | | | | |
| Screened case | | | | | 356 |
| Enrolled case | 62 | 63 | 62 | 62 | 249 |
| Completed case | 46 | 44 | 45 | 36 | 171 |
| FAS | 62 | 63 | 62 | 62 | 249 |
| PPS | 48 | 46 | 48 | 50 | 192 |
| SS | 62 | 63 | 62 | 62 | 249 |

| | | | | | |
|---|---|---|---|---|---|
| FAS: Full Analysis Set, refers to the set of eligible cases and dropped cases, but does not include excluded cases PPS: cases that meet the test protocol, have good compliance, and complete the connects filled according the CRF requirements SS: cases that receives at least one treatment and have actual data recorded by safety indicators | | | | | |

### V. Endpoint Index

The evaluation methods of clinical trial effect are as follows:

**Table 5**

| | | | | |
|---|---|---|---|---|
| Primary endpoint indicators and evaluation time | No | Indicator | Evaluation time | Selection of the endpoint indicator |
| | 1 | SLE response index at 12 months after administration. SLE response is defined as follows: | Week 48 | Effectiveness index |
| | | a. SELENA-SLEDAI score drop ≥ 4 points; | | |
| | | b. no new organs reach A grade in the BILAG score, or new organs do not exceed one B grade | | |
| | | c. the physician's global assessment (PGA) does not increase by more than 0.3 points from the baseline. The physician's global assessment uses a 100mm visual analog scale (VAS) to assess the comprehensive disease status before and after medication | | |
| Secondary endpoint indicators and evaluation time | No | Indicator | Evaluation time | Selection of the endpoint indicator |
| | 1 | (1) the proportion of subjects with SELENA-SLEDAI score decreased by ≥4 points after administration. (2) The change in the physician's global assessment score from baseline after administration. (3) The proportion of subjects with prednisone dose ≤7.5 mg/d or a decrease of dose ≥25% from baseline at 44-48 weeks after administration. (4) The change in serological test index (IgG, IgA, IgM, total B cell (CD 19+), anti-dsDNA antibodies, complement (C3, C4) from baseline | Week 48 | Effectiveness index |

### VI. Results of clinical trials

The clinical trial data analyzed by FAS (full analysis set) shows that the treatment group (TACI-Fc fusion protein: SEQ ID NO: 1) has a significantly higher response. The specific data are as follows:

**Table 6**

| Therapeutic doses | Efficacy at week 48 (SRI4) |
|---|---|
| 240 mg group | 75.8%, p <0.0001 |
| 160 mg group | 68.3%, p = 0.0001 |
| 80 mg group | 71.0%, p <0.0001 |
| Control group | 33.9%, p <0.0001 |

The SELENA-SLEDAI score at week 48 shows that more patients in the treatment group (TACI-Fc fusion protein: SEQ ID NO.1) has a reduction of 4 points or more (SELENA-SLEDAI score). The details are as follows: 80 mg dose group (75.8%, p = 0.003), 160 mg dose group (77.8%, p = 0.001), 240 mg dose group (79.0%, p <0.001), and placebo group (50.0%).

No more patients are found to have worsened PGA scores, as follows: 80 mg dose group (96.8%, p <0.001), 160 mg dose group (92.1%, p = 0.013), 240 mg dose group (96.8%, p <0.001) ), and placebo group (75.8%).

The analysis results from PPS (Per protocol set) and FAS (full analysis set) are consistent.

The data on clinical adverse events (AEs) and serious adverse events (SAEs) are as follows:

**Table 7. Incidence of AEs and SAEs**

| Group | Incidence of AEs | Incidence of SAEs |
|---|---|---|
| 240 mg group | 93.5% | 12.9% |
| 160 mg group | 92.1% | 15.9% |
| 80 mg group | 90.3% | 12.9% |
| Placebo group | 82.3% | 16.1% |

In the 240 mg group, there is one death unrelated to the test drug. The most common adverse events are upper respiratory tract infections and injection site reactions.

In addition, during clinical trials, there are 11 unexpected pregnancies in the TACI-Fc (SEQ ID NO.1) treatment group (4 cases in the 240 mg dose group, 3 cases in the 160 mg dose group, and 4 cases in the 240 mg dose group), while no pregnancy event in the placebo group. Of the 11 pregnancies, 10 cases chose selective termination and 1case chose pregnancy and live birth.

Therefore, the three dose groups of recombinant TACI-Fc fusion protein (SEQ ID NO.1) achieve very significant therapeutic effects and are safe and effective as proved by clinical data. The administrations for patients in the treatment group are well tolerated, and the ability to conceive of some patients may be unexpectedly restored by treating the patients with systemic lupus erythematosus.

## Claims

1. A method for treating systemic lupus erythematosus (SLE), comprising administering to a subject in need thereof a therapeutically effective amount of a pharmaceutical preparation of a recombinant TACI-Fc fusion protein in a dose greater than or equal to 50 mg per administration, wherein the recombinant TACI-Fc fusion protein comprises amino acids 13-118 of extracellular domain of TACI and the Fc region of human immunoglobulin.

2. The method of claim 1, wherein the dose is greater than or equal to 60 mg per administration, or 70 mg per administration, or 80 mg per administration.

3. The method of claim 1, wherein the dose is about 50-240 mg per administration, 60-240 mg per administration, 70-240 mg per administration, or 80-240 mg per administration, and preferably, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 190 mg, 195 mg, 200 mg, 205 mg, 210 mg, 215 mg, 220 mg, 225 mg, 230 mg, 235 mg, or 240 mg per administration.

4. The method of any one of claims 1 to 3, comprising administering the subject in need thereof once every week, every two weeks, every three weeks, or every four weeks.

5. The method of claim 4, comprising administering the subject in need thereof once every week.

6. The method of claim 3 or 5, wherein the pharmaceutical preparation is a lyophilized preparation or a liquid preparation.

7. The method of claim 6, wherein the pharmaceutical preparation is a lyophilized preparation.

8. The method of claim 6, wherein the pharmaceutical preparation is administered by subcutaneous injection, intraperitoneal injection, intramuscular injection, or intravenous injection, and preferably by subcutaneous injection.

9. The method of claim 8, wherein the pharmaceutical preparation is administered by subcutaneous injection.

10. The method of any one of claim 3 or 5 or 7 or 9, wherein the pharmaceutical preparation is administered to the subject in need thereof for consecutive 12 weeks, 24 weeks, 36 weeks, 48weeks, 60 weeks, 72 weeks or more.

11. The method of claim 10, wherein the pharmaceutical preparation is administered to the subject in need thereof for consecutive 48 weeks or longer.

12. The method of any one of claims 1-11, wherein two of the recombinant TACI-Fc fusion proteins form a double-stranded structure through interchain disulfide bond formed in Fc hinge region in a liquid pharmaceutical preparation.

13. The method of claim 12, wherein the amino acids 13-118 of the extracellular domain of TACI has a sequence as shown in amino acids 1-106 of SEQ ID NO: 1, or has 80%, 85%, 90%, 91%, 92%, 93%, 94%, 95%, 96%, 97%, 98%, 99% or more homology with amino acids 1-106 of SEQ ID NO: 1.

14. The method of claim 13, wherein the Fc region of human immunoglobulin has a sequence as shown in amino acids 107-333 of SEQ ID NO: 1, or has 80%, 85%, 90%, 91%, 92%, 93%, 94%, 95%, 96%, 97%, 98%, 99% or more homology with amino acids 107-333 of SEQ ID NO: 1.

15. The method of claim 14, wherein the recombinant TACI-Fc fusion protein has a sequence as shown in SEQ ID NO: 1.

16. The method of any one of claims 1-15, wherein the systemic lupus erythematosus is mild to moderate systemic lupus erythematosus or moderate to severe systemic lupus erythematosus.

17. A recombinant TACI-Fc fusion protein, comprising an amino acid sequence as shown in SEQ ID NO: 1.

18. A nucleotide sequence, encoding the amino acid sequence of recombinant TACI-Fc fusion protein as shown in SEQ ID NO: 1.

19. A vector, being capable of expressing the fusion protein of claim 17 or comprising the nucleotide sequence of claim 18.

20. A pharmaceutical composition, comprising the recombinant TACI-Fc fusion protein of claim 17 and a pharmaceutically acceptable carrier.

21. The pharmaceutical composition of claim 20, wherein the pharmaceutical composition is a liquid preparation.

22. The pharmaceutical composition of claim 21, wherein two of the TACI-Fc fusion proteins form a double-stranded structure through interchain disulfide bond formed in the Fc hinge region in the pharmaceutical composition.

23. The pharmaceutical composition of any one of claims 20-22, wherein the pharmaceutical composition is manufactured into a preparation containing 80-240 mg of the recombinant TACI-Fc fusion protein per unit.

24. The pharmaceutical composition of claim 23, wherein the pharmaceutical composition is a lyophilized preparation or a liquid preparation, and is administered by subcutaneous route in a dose of about 80-240 mg per administration.

25. The pharmaceutical composition of claim 24, wherein the pharmaceutical composition is used for the treatment of systemic lupus erythematosus, rheumatoid arthritis, neuromyelitis optica, and neuromyelitis optica spectrum disorders.

26. Use of the recombinant TACI-Fc fusion protein of claim 17 or the pharmaceutical composition of any one of claims 20-24 in the manufacture of a medicament for the treatment of autoimmune diseases.

27. The use of claim 26, wherein the autoimmune diseases include systemic lupus erythematosus, rheumatoid arthritis, neuromyelitis optica, and neuromyelitis optica spectrum disorders.
